# EUROPEAN PATENT APPLICATION

(11) **EP 1 364 648 A1**
(43) Date of publication of application: **26.11.2003**
(21) Application number: 01273578.3
(22) Date of filing: 23.10.2001
(51) Int. Cl.: A61K 31/343, A61P 25/28

(54) **CRYPTOTANSHINONE FOR PREVENTING AND ALLEVIATING ALZHEIMER'S DISEASE**

(30) Priority: 16.01.2001 CN 01107460
(71) Applicant: Zhongshan University, Guangdong 5102075 (CN); Guangzhou Medtech Zhongda Biotechnology Company, Ltd., Guangzhou Guangdong 5100080 (CN)
(72) Inventor: GU, Lianquan, Guangzhou Guangdong 510275 (CN); BU, Xianzhang, Guangzhou Guangdong 510275 (CN); LI, G. Guangzhou Medtech Zhongda Biotechn. Comp., Dongshan Dist. Guanzhou Guangdong 510275 (CN); MA, Lin, Guangzhou Guangdong 510275 (CN)
(74) Representative: Goddar, Heinz J., Dr.
(86) International application number: CN0101497
(87) International publication number: WO02060435

(57) **Abstract**

The present invention relates to use of Cryptotanshinone represented by formula (1): 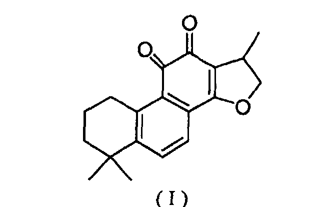 or a pharmaceutical composition containing the compound for manufacturing drug in preventing and treating early stage AD.

## Description

### Field of the Invention

This invention relates to use of Cryptotanshinone in manufacturing drugs for preventing and treating early stage Alzheimer's disease and method of using Cryptotanshinone to prevent and treating early stage Alzheimer's disease.

### Background of the Invention

Alzheimer's disease (AD) is a kind of progressive neuro-degenerative disease. Its major clinical symptoms are reduction of central recognition ability, deterioration of intelligence and memory, which result in self-care disability. According to statistic data, 5-10% aged people older than 60 and 24-40% people older than 80 have different degrees of AD symptoms. Therefore, AD is currently a common disease, which affects elder people's health seriously.

Characteristics of AD patients' pathological anatomy findings are coagulation and deposition of β-amyloid (Aβ-protein) and neurofibrillary tangle (NFT) in brain tissue. Causes of AD are very complicated. The cause of its pathogenesis is still not completely clarified through studies. Study results confirmed that all AD patients have abnormal symptoms as increase of blood ammonia level and intra-brain tissue ammonia concentration. Since ammonia has the greatest toxicity toward brain tissue, scientist thought that brain tissue long time surrounded by high-ammonia-concentration environment is the direct or indirect cause of development and deterioration of AD patients' symptoms (F. Michael, et al., Hyperammonemia in Alzheimer's Disease, Am J Psychiatry, 1985, 142, 71; S. Brain, et al., Elevated adenosine monophosphate deaminase activity in Alzheimer's disease brain, Neurobiology of Aging, 1998, 19, 385).

Blood ammonia in human body is mainly come from ammonia producing in intestine and kidney. When liver function is normal, most portion of ammonia produced by intestine or kidney can be detoxicated by urea synthesis through intra-hepatic Ornithine cycle. Chronic hepatitis and cirrhosis patients' liver function is impaired with ammonia-toxin detoxication ability decreased, therefore amount of ammonia infiltrated into blood circulation increased. Thus, chronic hepatitis and cirrhosis patients often have hyperammonemia symptoms. Ammonia is one kind of basic toxic substances, which has poisonous effect on most major organs of human body.

Ammonia in AD patients' brain tissue comes from 2 main routes: first one is that ammonia in blood enters brain tissue through Blood-brain-barrier; second route is that activity of some certain deaminase in AD patients' brain tissue is abnormal, such as activity of AMP deaminase is 2 times of control, which results in deaminase ability of adenine base of AMP increases and cause ammonia concentration in brain tissue elevates in turn. Since there isn't detoxication mechanism of ammonia in brain tissue, ammonia may remain in brain tissue for a long time and cause severe toxic damages to brain tissue and cause neuro-behavior injury, which is the main reason causing deterioration and aggravation of AD's symptoms (S. Nikolaus, An ammonia hypothesis of Alzheimer disease, in Advances in experimental medicine and biology, Vol 420, p235, Plenum Publishing Co., New York, 1997).

Still now, there are few drugs having effective treatment effect towards AD, therefore, one important method is to carry out prevention countermeasures against AD and to treat AD patients at the early stage. Use of drugs which can effectively eliminate hyperammonemia and high-level brain tissue ammonia is an effective way to prevent AD and treat early stage AD patients.

The present inventors have already found that Cryptotanshinone isolated from Dan-shen root, a Traditional Chinese Medicine, possesses excellent ability of eliminating hyperammonemia (PCT application No.: PCT/CN01/00861).

Dan-shen root is the root and rhizome of *Salvia Miltiorrhiza Bungev,* family labiatae; bitter in taste and attributive heart and liver channels. Dan-shen is widely used as a traditional Chinese herb. Its main indication is to disperse blood stasis and alleviate pain, to promote blood circulation and open channels, to clear away heat and relieve vexation, and so on. But till now, there are no reports on using extracts of dan-shen, such as Cryptotanshinone I, to prevent and treat hyperammonemia and high-level of brain ammonia. There also are no reports on using extracts of dan-shen, such as Cryptotanshinone I, to prevent AD and to treat early stage AD patients.

The object of this invention is to find and develop the new pharmaceutical use of Cryptotanshinone I in the dan-shen extracts.

Through investigation, the inventors found that Cryptotanshinone I extracted from dan-shen can effectively lower the blood ammonia concentration and brain tissue ammonia concentration, which indicates that Cryptotanshinone possesses a bright future in prevention and treating early stage AD patients.

This invention provide a new drug used in preventing AD and treating early stage AD patients, and the drug is developed on the basis of mechanism that Cryptotanshinone can effectively eliminate hyperammonemia and that Cryptotanshinone can enter into brain tissue through blood-brain-barrier, thereby effectively eliminating high-concentration ammonia in the brain tissue.

### Summary of the Invention

This present invention provides the use of Cryptotanshinone compound represented by formula (I): 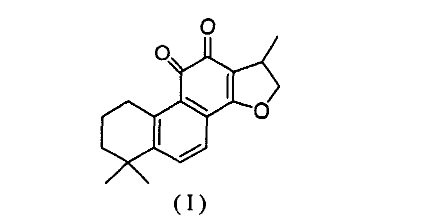 or a pharmaceutical composition containing the compound for manufacturing drug in preventing and treating early stage AD.

This present invention further provides a pharmaceutical composition for preventing and treating early stage AD. The pharmaceutical composition contains Cryptotanshinone compound represented by formula (I) and optionally a pharmaceutically acceptable diluent or vehicle.

This present invention further provides a method for preparing a pharmaceutical composition for preventing AD and treating early stage AD patients, which comprises formulating Cryptotanshinone compound represented by formula (I) optionally with a pharmaceutical acceptable diluent or vehicle.

This present invention further provides a method for preventing AD and treating early stage AD patients, which comprises administering to the patient an effective amount of Cryptotanshinone compound represented by formula (I) or a pharmaceutical composition containing the same.

Cryptotanshinone represented by formula (I) in this invention can achieve the aim of prevention and delay early stage AD by passing through the blood-brain-barrier, entering into the brain tissue and eliminating abnormal high concentration ammonia in the brain tissue of the early stage AD patients.

According to this invention, Cryptotanshinone represented by formula (I) and its pharmaceutical composition can be prepared by the method well-known in the arts and can be delivered through oral, parenteral (such as intravenous injection, intra-muscular injection, hypodermic injection etc.) or topical (such as sublingual or rectal etc.) administration. Oral preparations include the form of tablets, chewing tablets, capsule, pill, suspension, emulsion, solution and so on. Parenteral preparations include injection, topical administration preparations include formulations such as cream, ointment, sticking, suppository and spray etc.

Cryptotanshinone compound represented by formula (I) or its pharmaceutical composition containing the compound provided by this invention is a kind of drug for preventing AD and treating early stage AD patients with good safety.

### Detailed Description of the invention

Research results of the inventors showed that Cryptotanshinone can react with ammonia under a mild condition to form new tanshinonic derivatives. Cryptotanshinone can also initiate similar reactions with other toxic amines substance, such as phenylethylamine, in brain tissue. It suggests that this compound possesses the function of eliminating the blood ammonia at high concentration and the brain ammonia at high level. This shows that this compound can be used in treating hyperammonemia and high level brain ammonia and possesses unique function of preventing AD and treating early stage AD patients. The reaction formula is illustrated below:

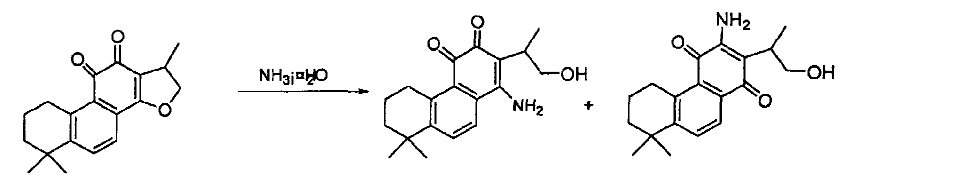

Cryptotanshinone used in this invention can be isolated from Traditional Chinese Medicine Dan-shen by solvent extraction; it can also be obtained through chemical synthesis method. The extraction method is generally used to achieve the compounds in the present invention considering the factors of cost etc. for the abundant resources of dan-shen.

In the present invention, the inventors confirmed through chemical reaction experiments that Cryptotanshinone could react with ammonia or amines to form tanshinone derivatives. It indicates that this compound can be used in lowering high concentration ammonia (amines) in blood and brain tissue.

The present invention demonstrated through hyperammonemia animal models experiments that Cryptotanshinone could effectively decrease blood ammonia concentration of hyperammonemia rat model. It indicates that Cryptotanshinone can be applied as effective drug used in preventing and treating hyperammonemia, in preventing AD and treating early stage AD patients.

Through high-level brain ammonia animal model experiment, this invention confirmed that Cryptotanshinone could effectively decrease ammonia concentration in rat's brain tissue. It shows that Cryptotanshinone can be used as an effective drug for preventing and treating high level brain ammonia, thereby preventing AD and treating early stage AD patients.

The present invention demonstrates, through AD neuro-behavior experiments in animal models, that Cryptotanshinone could effectively delay model rat's injured neuro-behavior time and improve model rat's injured neuro-behavior symptoms. It indicates that Cryptotanshinone can be used as an effective drug for preventing AD and treating early stage AD patients.

The present invention determined through toxicologic study in rats that Cryptotanshinone is one kind of drug without toxic and adverse effects and can be used safely.

The inventors found that the content of Cryptotanshinone is highest in blood and brain tissue through metabolism experiments in rats. This indicates that Cryptotanshinone can be easily absorbed and entered into blood circulation. Since Cryptotanshinone is liposoluble small molecular compound (molecular weight 296 Da), it can pass blood-brain-barrier and enter into brain tissue easily. This feature of Cryptotanshinone is the important reason of its role as effective drugs in preventing and treating hyperammonemia and high brain tissue ammonia concentration situation, and in preventing AD and treating early stage AD patients (M.P. William, Crossing the blood-brain barrier: are we getting it right? *Drug Discovery Today.* 2001.6.1).

Drugs currently used in treating hyperammonemia are mainly lactulose, sodium glutamate and arginine [DRUG; Wang Rulong, Yuan Zhengping, Chemistry Industry Publishing House, 3^{rd} Edition (1999), 655-665], but they all have limitation as well as dissatisfactory treating effect. By now, there is no report on the drug which can pass blood-brain-barrier and eliminate high concentration ammonia in brain tissue with an excellent safety.

Followings are examples further describing the present invention.

### Example 1

Extraction, isolation and purification of Cryptotanshinone from Dan-shen:

Dried dan-shen 1 kg was extracted 3 times 24 h each using 1500 mL 95% alcohol, then vacuum concentrated to 500 ml, and added 500 ml water, then extracted 4 times using 1000 ml trichloromethane, then the extract was vacuum concentrated, and isolated through gradient elution by column chromatography, with silica gel of 100-200 mesh, and eluent as petroleum ether / acetic ester mixture containing 1%-50% acetic ester. About 0.35g Cryptotanshinone obtained.

### Example 2

Cryptotanshinone's in vitro reaction with ammonia:

0.5 mmol ammonia and 30 ml water was added into a tube, and then added 0.5 mmol Cryptotanshinone in 1.0 ml alcohol, and vibrated 0.5-3 h in 37±5°C, then monitored by TLC and isolated by column chromatography, and obtained the products of Cryptotanshinone and ammonia reaction as 1-amino-2-(1-hydroxy-2-propyl)-8,8-dimethyl-5,6,7,8-tetrahydrophenanthrene-3,4-dione and 3-amino-2-(1-hydroxy-2-propyl)-8,8-dimethyl-5,6,7,8-tetrahydrophenanthrene-1,4-dione. Structure of reaction products have already been confirmed by data of ¹³C-¹HNMR, MS and elements analysis. This indicates that Cryptotanshinone can react with ammonia and lower its concentration in vitro.

### Example 3

In vitro interaction of Tanshinone IIA (another type of tanshinone compounds in dan-shen) with ammonia:

No apparent reaction showed in 48h monitoring with TLC according to the method of Example 3 and substituting Cryptotanshinone with tanshinone IIA, which suggested that no interaction exists in this condition between tanshinone IIA and ammonia.

### Example 4

Blood ammonia reducing experiment of Cryptotanshinone and Tanshinone IIA(in injection):

The animals used in this experiment were 50 healthy male SD rats weighing 250-300g. They were randomly divided into 5 groups, that is, normal control group, experimental control group (acetamide group), sodium glutamate/acetamide group, Cryptotanshinone /acetamide group and tanshinone IIA/acetamide group. The rats in all those groups except the normal control group were injected intraperitoneally with saline, sodium glutamate injection (with a dose of 410 mg/kg), saline solution of Cryptotanshinone (containing small amount of surface active agent, with a dose of 10 mg/kg), saline solution of tanshinone IIA (containing small amount of surface active agent, with a dose of 10 mg/kg) respectively. 45 min after the injection, the rats in all those groups except normal control group were injected intraperitoneally with 5.5 mmol/kg acetamide. Above injections continued for 4 days. In the 4^{th} day, 30 min after the acetamide injection, blood samples of the rats in each group were collected by eyeball removing method immediately, and processed with the anticoagulant EDTA-Na, then the blood ammonia concentration was determined using conventional method. The results were showed in Table 1.

The dose of sodium glutamate in this example and following examples was settled with the reference of its actual clinical dose. The method for determining the blood ammonia concentration in this example and following examples was enzyme-UV method, the instrument used was HITACHI-7170 automatic analyzer, and the kit used was AMMONIA kit. Calculation formula of decrease ratio in Table 1 and following tables was: Decrease rate = [ 1-(concentration of treating group - concentration of normal control group) / (concentration of experimental control group - concentration of normal control group)]×100%.

**Table 1**

| Groups | Blood ammonia concentration (□ mol/L) | Reduction rate (%) |
|---|---|---|
| Normal control group | 115 | --- |
| Experimental control group | 885 | --- |
| Sodium Glutamate group | 480 | 53 |
| Cryptotanshinone group | 420 | 60 |
| Tanshinone IIA group | 850 | <5 |

Using the same experiment method, 90 min after the acetamide injection at the 4^{th} day, blood samples of the rats in each group were collected by eyeball removing method immediately. The blood ammonia concentration was determined. The results were showed in Table 2.

**Table 2**

| Groups | Blood ammonia concentration (□ mol/L) | Reduction rate (%) |
|---|---|---|
| Normal control group | 115 | --- |
| Experimental control group | 285 | --- |
| Sodium Glutamate group | 120 | 97 |
| Dihydrotanshinone I group | 110 | 100 |
| Tanshinone IIA group | 265 | <12 |

### Example 5

Blood ammonia reducing experiment of Cryptotanshinone and tanshinone IIA (in oral):

The same experiment condition in example 4 was adopted except Sodium Glutamate oral taking (the dose was 200 mg/kg) instead of Sodium Glutamate injecting, Cryptotanshinone oral taking (the dose is 100 mg/kg) instead of injecting, and tanshinone IIA oral talking (the dose is 100 mg/kg) instead of injecting. In the 4^{th} day, 30 min after the acetamide injection, blood samples of the rats in each group were collected by eyeball removing method immediately, and processed with the anticoagulant EDTA-Na, then the blood ammonia concentration was determined. The results were shown in Table 3.

**Table 3**

| Groups | Blood ammonia concentration (□ mol/L) | Reduction rate (%) |
|---|---|---|
| Normal control group | 115 | --- |
| Experimental control group | 885 | --- |
| Sodium Glutamate group | 510 | 49 |
| Cryptotanshinone group | 470 | 54 |
| Tanshinone IIA group | 865 | <4 |

Using the same experiment method, 90 min after the acetamide injection at the 4^{th} day, blood samples of the rats in each group were collected by eyeball removing method immediately. The blood ammonia concentration was determined. The results were showed in Table 4.

**Table 4**

| Groups | Blood ammonia concentration (□ mol/L) | Reduction rate (%) |
|---|---|---|
| Normal control group | 115 | --- |
| Experimental control group | 285 | --- |
| Sodium Glutamate group | 130 | 91 |
| Dihydrotanshinone I group | 120 | 97 |
| Tanshinone IIA group | 270 | <10 |

### Example 6

Brain tissue ammonia concentration reducing experiment of Cryptotanshinone (injection):

Experimental animals were 50 healthy male SD rats, weight between 250-300g. They were randomly divided into 4 groups, i.e. normal control group, experimental control group (ammonium acetate group), sodium glutamate/acetamide group, and Cryptotanshinone/ acetamide group. The rats in all those groups except the normal control group were injected intraperitoneally with saline, sodium glutamate solution (with a dose of 410 mg/kg), and saline solution of Cryptotanshinone (containing small amount of surface active agent, with a dose of 30 mg/kg) respectively. 45 min after the injection, the rats in all those groups except the normal control group were injected intraperitoneally with 5.5 mmol/kg acetamide. Above injections continued for 4 days. In the 4^{th} day, 60 min after the acetamide injection, the rats of each group were put to death, and brain tissue samples were taken out immediately and homogenized under low temperature condition, then the ammonia concentration in brain tissue was determined by using glutamate dehydrogenase assay. The results were listed in Table 5.

**Table 5**

| Groups | Brain tissue ammonia concentration (nmol/g) | Reduction rate (%) |
|---|---|---|
| Normal control group | 190 | --- |
| Experimental control group | 410 | --- |
| Sodium Glutamate group | 350 | 28 |
| Cryptotanshinone group | 280 | 60 |

### Example 7

Brain tissue ammonia concentration reducing experiment of Cryptotanshinone (oral):

The same experiment condition in example 6 was adopted except Sodium Glutamate oral taking (the dose was 200 mg/kg) instead of Sodium Glutamate injection and Cryptotanshinone oral taking (dose 150 mg/kg) instead of Cryptotanshinone injection. In the 4^{th} day, 60 min after the acetamide injection, the rats of each group were put to death and brain tissue samples were taken out immediately. Then, the ammonia concentration in brain tissue was determined. The results were listed in Table 6.

**Table 6**

| Groups | Brain tissue ammonia concentration (nmol/g) | Reduction rate (%) |
|---|---|---|
| Normal control group | 190 | --- |
| Experimental control group | 410 | --- |
| Sodium Glutamate group | 370 | 19 |
| Cryptotanshinone group | 310 | 45 |

### Example 8

Impaired Neuro-behavioral symptoms relieving experiments of Cryptotanshinone in AD rat model:

Experimental animals were 40 healthy male SD rats, weight between 250-300g. They were randomly divided into 4 groups, i.e. normal control group, model control group A (D-galactose/ quinolinic acid group), model control group B (D-galactose/ acetamide group), and Cryptotanshinone group (D-galactose / acetamide /Cryptotanshinone group). Morris water labyrinth method was used to evaluate the neuro-behavioral symptoms of rats model caused by subacute senium and chemical damage by D-galactose.

Normal control group: routine breeding.

Model control group A (D-galactose/quinolinic acid group): D-galactose was injected subcutaneously (50 mg/kg/day, for 6 weeks) to cause subacute senium in rats. Quinolinic acid (1 µL, containing quinolinic acid 75 nmol) was injected into the rats' bilateral hippocampal gyrus to evidently decrease rats' labyrinth learning and memorizing ability, which served as animal model similar to AD.

Model control group B (D-galactose/ acetamide group): D-galactose was injected subcutaneously (50 mg/kg/day, for 6 weeks) to cause subacute senium in rats; acetamide (10 mmol/kg/day) was injected intraperitoneally for 7 continuous days. Hyperammonemia resulted in high ammonia level in brain tissue and caused damage to brain tissue, thereby evidently decreasing rats' labyrinth learning and memorizing ability. The treated rats were served as animal model similar to AD.

Cryptotanshinone group (D-galactose / acetamide /Cryptotanshinone group): Simultaneously with subcutaneous injection of D-galactose (50 mg/kg/day, for 6 weeks), Cryptotanshinone was orally administered (the dose was 200 mg/kg/day). 10 mmol/kg acetamide was intraperitoneally injected for 7 continuous days (Cryptotanshinone was orally given at the same time). The results of Morris water labyrinth test of rats in each group were listed in Table 7.

**Table 7**

| Group | AD Symptom-Amnesia Synthetic Index (Scored by day after chemical damage) | | | | | | |
|---|---|---|---|---|---|---|---|
| | Day 1 | Day 2 | Day 3 | Day 4 | Day 5 | Day 6 | Day 7 |
| Normal control group | □ | □ | □ | □ | □ | □ | □ |
| Model control group A | ++++ | +++++ | +++++ | +++++ | +++++ | +++++ | +++++ |
| Model control group B | + | ++ | ++ | +++ | +++ | ++++ | ++++ |
| Cryptotanshinone Group | □ | □ | □ | □ | + | + | + |
| Note: □ Normal; + Amnesia (Number of + indicates the extent of memory impairment, and +++++ represents the most severe case.) | | | | | | | |

### Example 9

Toxicity test of Cryptotanshinone:

Acute toxicity test in mouse: The animals used in this study were healthy male mice. The drug was administrated intragastrically. Still no toxic symptom or death showed in the experiment mice when the administrated dose added to 1.5 g/kg. The experiment showed that LD₅₀ of Cryptotanshinone > 30 g / kg.

30 days toxicity test in mouse: The animals used in this study were healthy male mice. The drug was continually administrated intragastrically for 30 days with dose of 200 mg/kg day. The results showed that there was no death of the tested mice, and no apparent abnormal change or toxic reaction in the growth and development, hematopoiesis function, and biochemical index observed in the tested mice. No pathology change of major organs was found by anatomical inspection and tissue microscopy inspection.

### Example 10

Brain tissue entrance experiment of Cryptotanshinone:

Experimental animals were healthy male rats. The drug was continually administrated intragastrically at a dose of 300 mg/kg/day, continued for 5 days. 1 hour after administration on the 5^{th} day, rats were put to death and brains were taken out. The brain tissues were homogenized and then extracted with chloroform. Significant amount of Cryptotanshinone was detected with HPLC.

### Industrial applicability

Above experiments of this invention indicate:
1) Cryptotanshinone represented by formula (I) in this invention can interact with ammonia or amines under physiological conditions and thereby eliminating ammonia or amines.
2) Experiments in hyperammonemia animal model showed that Cryptotanshinone represented by formula (I) in this invention possess excellent ability in reducing blood ammonia concentration.
3) Experiments in high brain tissue ammonia level animal models showed Cryptotanshinone represented by formula (I) in this invention possess excellent ability to reducing ammonia level in brain tissue.
4) Neuro-behavioral experiments in AD animal model showed that Cryptotanshinone represented by formula (I) in this invention can effectively delay AD rats' impaired neuro-behavior time and relieve impaired neuro-behavior symptoms of model rats.
5) Toxicological test with Cryptotanshinone represented by formula (I) in this invention showed that Cryptotanshinone is safely used as drug under normal dosage.
6) Cryptotanshinone represented by formula (I) in this invention can be absorbed into blood by GI tract and therefore eliminates blood ammonia. Simultaneously, Cryptotanshinone can pass blood brain barrier and enter into brain tissue, thereby ammonia in brain tissue can be eliminated.
7) Compared with glutamic acid which is a typical drug commonly used to treat hyperammonemia in the art, the same or more effective result can be obtained from the compound in the present invention with even smaller dosage. The therapeutic efficacy of the compound in the present invention is more significant than glutamic acid especially when the liver function is damaged.
8) Because Glutamic acid cannot pass blood brain barrier, it has little effect of reducing brain ammonia level. Whereas Cryptotanshinone can pass blood brain barrier and enter into brain tissue, so it possesses excellent function of eliminating high-level brain ammonia.
9) Cryptotanshinone represented by formula (I) in this invention can pass blood brain barrier and enter into brain tissue; it possesses excellent function of elimination high-level brain ammonia. This indicates that it can be used to prevent and treat high-level brain ammonia, and served as effective drugs to prevent AD and treat early stage AD patients.

## Claims

1. Use of Cryptotanshinone compound represented by formula (I): 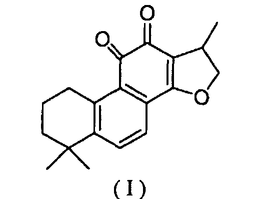 or a pharmaceutical composition containing the compound for manufacturing drug in preventing and treating early stage AD.

2. The use of claim 1, wherein Cryptotanshinone represented by formula (I) can achieve the aim of prevention and delay early stage AD by passing through the blood-brain-barrier, entering into the brain tissue and eliminating abnormal high concentration ammonia in the brain tissue of the early stage AD patients.

3. A pharmaceutical composition for preventing and treating early stage AD, comprising Cryptotanshinone compound represented by formula (I) and optionally a pharmaceutically acceptable diluent or vehicle.

4. A method for preparing a pharmaceutical composition for preventing AD and treating early stage AD patients, comprising formulating Cryptotanshinone compound represented by formula (I) optionally with a pharmaceutical acceptable diluent or vehicle.

5. A method for preventing AD and treating early stage AD patients, comprising administering to the patient an effective amount of Cryptotanshinone compound represented by formula (I) or a pharmaceutical composition containing the same.

6. The use of claims 1 or 2, wherein said medicine is administered in a form selected from the group consisting of injection, tablet, pill, capsule, solution, suspension and emulsion.

7. The use of claims 1 or 2, wherein said medicine is administered to a patient by a route selected from the group consisting of oral, sublingual, intravenous, intra-muscular, subcutaneous and rectal.
